# EUROPEAN PATENT APPLICATION

(11) **EP 4 825 311 A1**
(43) Date of publication of application: **21.10.2026**
(21) Application number: 25170261.9
(22) Date of filing: 14.04.2025
(51) Int. Cl.: A61B 5/01, G01K 13/20, G01K 7/42

(54) **CORE BODY TEMPERATURE ESTIMATION**

(71) Applicant: Koninklijke Philips N.V., 5656 AE Eindhoven (NL)
(72) Inventor: HILGERS, Achim Rudolf, 5656 AG Eindhoven (NL); MUEHLSTEFF, Jens, 5656 AG Eindhoven (NL)
(74) Representative: Philips Intellectual Property & Standards

(57) **Abstract**

The present invention relates to a core body temperature estimation device, a core body temperature estimation system and a core body temperature estimation method for estimating a user's core body temperature and to a sensor device for measuring optical and thermal features of a user's skin- and/or subcutaneous tissue. The core body temperature estimation, CBT, device for estimating a user's CBT comprises an input unit configured to obtain first temperature data comprising information about a skin- and/or subcutaneous tissue temperature of the user, second temperature data comprising information about a second temperature, and optical data comprising information about an optical transmission and/or absorption of the user's skin- and/or subcutaneous tissue; a processing unit configured to estimate a thickness of the user's skin- and/or subcutaneous tissue based on the optical data and to estimate the user's CBT based on the first temperature data, the second temperature data and the estimated thickness, and an output unit configured to provide CBT information comprising information about the estimated CBT.

## Description

### FIELD OF THE INVENTION

The present invention relates to a core body temperature estimation device, a core body temperature estimation system and a core body temperature estimation method
for estimating a user's core body temperature and to a sensor device for measuring optical and thermal features of a user's skin- and/or subcutaneous tissue.

### BACKGROUND OF THE INVENTION

Core body temperature (CBT), also called core temperature, is the operating temperature of an organism, specifically in deep structures of the body such as the liver, brain and heart. In other words, CBT mirrors the temperature of the center of the body. Core body temperature is normally maintained within a narrow range so that essential homeostasis is not disturbed. In healthy subjects it changes comparably within a small region from 36.5 °C to 37.5 °C.

When the body heats up because of external heating sources, internal mechanisms cool the body down to ensure the body functions at its best. Similarly, when the external environment becomes colder than the organism, internal processes heat the body up, in order to ensure a very dynamic protection mechanism of the body.

Core body temperature is also correlated to temperatures of peripheral tissues, such as skin and other surface areas. The peripheral temperatures fluctuate much more due to changing environmental circumstances. For example, oral temperature is affected by hot or cold drinks and mouth-breathing thus estimating the CBT from the oral temperature can be inaccurate. Significant core temperature elevation "hyperthermia" or depression "hypothermia" can cause serious life-threatening problems.

Inside an operation room, core body temperature, along with blood pressure, heart rate, breath rate, form the four basic essential vital signs of evaluating the user's condition. As a vital indicator for doctors, continuous monitoring of core temperature plays an important role in assisting to act during surgery, especially when a user is undergoing anesthesia. Unintended hypothermia can develop immediately following the induction of anesthesia. For a user undergoing anesthesia, the body's metabolism automatically drops and also the thermal regulatory system gets inhibited. As a result, the body temperature decreases, which can lead to hypothermia, which then could result in additional adverse events. The user's core body temperature may also be monitored to detect changes in the body temperature (hypo- or hyperthermia) to guide thermal management as well as to detect fever caused e.g. by an infection.

In a clinical environment CBT is measured most precisely by means of putting a temperature probe close to the body center (e.g. through the trachea, the esophagus, or the pulmonary artery). However, efforts have been spent on inferring CBT conducted on dedicated spots on the (surface of the) human body via heat flux measurements from the skin. Typical sides are spots where an artery is very close beneath the skin, like e.g. the forehead. However, depending on the actual implemented CBT-technology, a solution can become complex and costly if a certain precision is required, or usually suffers in precision due to uncertainties regarding environmental effects/changes and a few assumptions to be made.

In clinical practice, most preferable body temperature measurement techniques and their individual advantages and disadvantages are given by the following table (based on Hymczak et al., "Core Temperature Measurement - Principles of Correct Measurement, Problems, and Complications", International Journal of Environmental Research and Public Health, 2021, 18 (20, 10606):

| Measurement location | Accuracy | Special Requirements | Advantages | Disadvantages |
|---|---|---|---|---|
| Body surface | Poor | None | Easy and widely available (also in prehospital care) | Inaccurate |
| Oral | Poor | None | Easy and widely available (also in prehospital care) | Inaccurate |
| Axilla | Poor | None | Easy and widely available (also in prehospital care) | Inaccurate |
| Rectum | Good | Rectal probe | Easy, widely available (also in prehospital conditions), precise, and repeatable | High latency |
| Urinary Bladder | Good | Urinary catheter | Precise and repeatable | High latency |
| Tympanic membrane | Good | Special insulated Thermistor | Precise, repeatable, and brain core temperature (possible application in neurosurgery and deep hypothermia cardiac surgery) | High risk of measurement error |
| Nasopharynx | Good | None | Easy and widely available (also in prehospital conditions) | High risk of measurement error |
| Esophagus | Most precise | Long esophageal Thermistor | Easy, widely available (also in prehospital conditions), and repeatable | High latency |
| Pulmonary Artery | Most precise | Swan-Ganz catheter | Precise and repeatable | Invasive and restricted to intensive care units |

EP 3 666 179 A1 discloses a core body temperature sensor system which has multiple pairs of temperature sensors across a first insulating layer. The sensed temperatures are monitored as well as the rate of change of sensed temperature at the temperature sensors. The core body temperature is obtained based on a first representative temperature for the first set of temperature sensors, a second representative temperature for the second set of temperature sensors and rate of change information.

### SUMMARY OF THE INVENTION

It is an object of the present invention to improve CBT measurements, particularly CBT measurements based on heat flux methodology, to make them more precise and, in particular, to adapt them to a user-specific physiology.

In a first aspect of the present invention a core body temperature estimation device for estimating a user's CBT is presented, the CBT estimation device comprising:
- an input unit configured to obtain
   - first temperature data comprising information about a skin- and/or subcutaneous tissue temperature of the user (i.e. about a first temperature),
   - second temperature data comprising information about a second temperature (e.g. environmental temperature), and
   - optical data comprising information about an optical transmission and/or absorption of the user's skin- and/or subcutaneous tissue;
- a processing unit configured to estimate a thickness of the user's skin- and/or subcutaneous tissue based on the optical data and to estimate the user's CBT based on the first temperature data, the second temperature data and the estimated thickness, and
- an output unit configured to provide CBT information comprising information about the estimated CBT.

In a second aspect of the present invention a sensor device for measuring optical and thermal features of a user's skin- and/or subcutaneous tissue is presented, the sensor device comprising:
- a first temperature sensor configured to measure a skin- and/or subcutaneous tissue temperature of the user (i.e. a first temperature);
- a second temperature sensor configured to measure a second temperature (e.g. environmental temperature);
- an optical sensor configured to measure transmission and/or absorption (of optical radiation) of the user's skin- and/or subcutaneous tissue; and
- an insulating layer comprising a thermally insulating material,
wherein the insulating layer is (at least) arranged between the second temperature sensor and the first temperature sensor and configured to thermally isolate the first temperature sensor from the second temperature sensor.

In a third aspect of the present invention a core body temperature estimation method for estimating a user's CBT is presented, the method comprising:
- obtaining first temperature data comprising information about a skin- and/or subcutaneous tissue temperature of the user;
- obtaining second temperature data comprising information about a second temperature;
- obtaining optical data comprising information about transmission and/or absorption of the user's skin- and/or subcutaneous tissue;
- estimating a thickness of the user's skin- and/or subcutaneous tissue based on the optical data;
- estimating the user's CBT based on the first temperature data, second temperature data and the estimated thickness; and
- providing CBT information, comprising information about the estimated CBT.

In a further aspect of the present invention a core body temperature estimation system for estimating a user's CBT is presented, the system comprising:
a core body temperature estimation device as for estimating a user's CBT as presented herein; and
any one of the following:
   - an optical sensor configured to measure transmission and/or absorption (of optical radiation) of the user's skin- and/or subcutaneous tissue (and to provide optical data comprising information about the measured optical transmission and/or absorption of the user's skin- and/or subcutaneous tissue);
   - a first temperature sensor configured to measure skin- and/or subcutaneous tissue temperature of the user (and to provide first temperature data comprising information about the measured skin- and/or subcutaneous tissue temperature of the user); and
   - a second temperature sensor configured to measure a second temperature (and to provide second temperature data comprising information about the measured second temperature).

In yet a further aspect of the present invention, there is provided a corresponding computer program which comprises program code means for causing a computer to perform the steps of the CBT estimation method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, computer program and medium have similar and/or identical preferred embodiments as the claimed CBT estimation device and CBT estimation system, in particular as defined in the dependent claims and as disclosed herein.

The present invention is based on the idea to identify (measure) optical characteristics of the skin- and/or subcutaneous tissue of a user in order to derive a user-specific optical transfer function/parameter, to finally improve the precision of a measurement of the user's core body temperature.

In particular, the present invention is based on the idea to improve body temperature measurement techniques using temperature sensors which are positioned on the skin and correlate the skin- and/or subcutaneous tissue temperature to a certain core body temperature.

A general technique to identify a temperature is based on the heat flow/flux principle. In general, heat flows from a point with a higher temperature to a point with a lower temperature. Heat flow resistance (also known as thermal resistance) is a measure of a material's ability to resist the flow of heat. It quantifies how well a material or system prevents heat from passing through it. For a material being arranged between a first position with temperature *T*₁ and a second position with temperature *T*₀ the heat flow resistance *R* is defined as: $R = \frac{{T}_{0} - {T}_{1}}{Q / A} = \frac{l}{k} ,$ (1) wherein, *Q* is the energy / heat flowing through a cross-sectional area *A* of the material. *R* can likewise be defined to be a ratio between the distance between the first and second position and a material constant (to be more precise the thermal conductivity) *k* of the material between the first and second position.

The so called "single heat flux approach" is a method for performing temperature measurements of remote areas such as the core of a human body. The method requires two temperature sensors that are separated by an insulating material, wherein one of said sensors is configured to be arranged directly on the user's skin. When placed on the user's skin, heat from the body core flows through the tissue of the user's body and through the insulating material between both temperature sensors. If the temperature of the user's skin (and/or subcutaneous tissue) *Tₛₖᵢₙ* as measured by one of the sensors and another (second) temperature, e.g. the environmental temperature *Tₑₙᵥ* as measured by the other temperature sensor and both the heat flow resistance of the insulating material *Rₜᵣₐₙₛ* as well as the heat flow resistance of the user's body *R_{core}* are known, then core body temperature *T_{core}* can be calculated using the following equation: ${T}_{core} = {T}_{skin} + \frac{{R}_{core}}{{R}_{trans}} ⋅ \left({T}_{skin}-{T}_{env}\right) .$ (2)

It should be noted that in this approach, for simplicity reasons, lateral heat flow mechanisms can be neglected.

The relationship *R_{core}*/*Rₜᵣₐₙₛ* is assumed or inferred/defined by means of measurements of *Rₜᵣₐₙₛ* and/or a clinical study. The relationship is typically known as α (alpha), i.e. α *= R_{core}*/*Rₜᵣₐₙₛ.*

*R_{core} = l*/*k* depends on several parameters, particularly the physiological conditions of a user. E.g. a mid-age obese male user may have a significantly different heat transfer function *R_{core}* than a healthy and normal weight woman. In fact, *R_{core}* is location- and user-specific. Examples of relevant factors are:
- Tissue properties, including differences in fat-, protein- and water content, which is relevant of the parameter *k*,
- Position of an artery in relation to the temperature sensors (e.g. how deep below the skin an artery is located), which is relevant for the parameter *l*
- Sweat / skin excrements, building up under the temperature sensor for measuring skin temperature, which is relevant for both parameters *k* and *l*
- Changes in skin water content of the user's skin, which is relevant for both parameters k and*l*
- The quality of contact between the temperature sensor for measuring the skin temperature and the user's skin, which is relevant for both parameters *k* and *l.*

However, despite the significant impact on the precision of core body temperature estimation based on e.g. single heat flux measurement techniques, currently, a fixed value of *R_{core}* is assumed for CBT measurements. In particular, an alpha value of 0.301 has been predicted from lab experiments, while a theoretical approach leads to a value of α = 0.3110. Therefore, it is proposed to find a user specific approach which takes into account the above-mentioned user-specific features to improve core body temperature estimation.

Accordingly, it is proposed to measure the optical skin- and in particular the subcutaneous tissue characteristics of the user, preferably at the position where a CBT sensor will be placed, and to estimate the core body temperature by taking into account said measured characteristics, ultimately leading to an improved and user-specific CBT measurement.

As already explained, the thickness of the user's skin- and subcutaneous tissue is correlated with *R_{core}.* Thus, it is proposed to estimate the thickness of the user's skin- and/or subcutaneous tissues and to estimate the user's CBT based on the temperature of the user's skin- and/or subcutaneous tissue, the second temperature and the estimated thickness of the user's skin- and/or subcutaneous tissue. The second temperature may be given by the environmental temperature, i.e. the temperature of the (close) environment around the user. In general, it is preferred that the second temperature is independent from the first temperature (user temperature). The thickness is estimated using optical data comprising information about an optical transmission and/or absorption of the user's skin- and/or subcutaneous tissue.

Estimating/determining a user-specific thickness of the skin- and/or subcutaneous tissue allows for a more accurate CBT estimation, as it accounts for individual physiological differences in tissue structure. Accordingly, measurement errors that arise from generalized assumptions, particularly for users with non-standard body compositions (e.g., obese or underweight individuals) are reduced.

In an embodiment of the device, the processing unit is further configured to determine a user-specific heat transfer function based on the estimated thickness and to estimate the user's CBT based on the first temperature data, the second temperature data and the determined heat transfer function.

In other words, it is proposed to find a patient-specific (core body) heat transfer function to improve the core body temperature estimation. By measuring the optical skin- and/or subcutaneous tissue characteristics of the user (preferably at the position where the CBT sensor will be placed) an optical transfer function/parameter (specifically matching the before measured skin- and subcutaneous tissue characteristics) can be determined and thus also thickness of the user's skin- and/or subcutaneous tissue can be determined. The characteristic optical transfer function/parameter, i.e. the user-specific heat transfer function, can then be used to improve the precision of the CBT measurement of the CBT sensor.

In another embodiment, the processing unit is (further) configured to determine the heat transfer function based on a look-up table and/or a fitting function relating the estimated thickness to the heat transfer function.

This way, by using pre-calculated values a fast and efficient computation allowing real-time CBT estimation can be ensured. Both the look-up table and the fitting function may be optimized based on clinical studies or machine learning models to enhance accuracy over time.

In an embodiment, the relationship between the estimated thickness and the heat transfer function is a linear relationship.

The inventors used a measurement setup comprising a silicone layer of given thickness, mimicking a user's skin/tissue thicknesses, a heat plate that was set to have a given temperature (set point), mimicking the CBT, and a CBT sensor comprising a temperature sensor for measuring *Tₑₙᵥ* and a temperature sensor for measuring *Tₛₖᵢₙ* (i.e. the temperature of the silicone layer *T_{silicone}* mimicking the user's skin). When arranging the silicone layer on top of the heat plate and arranging the CBT sensor on top of the silicone layer, a simple averaged linear correlation was derived, approximating the alpha value as a function of the silicone thickness (mimicking user skin/tissue thickness) d: $α = 0.0782 ∗ d - 0.0017 .$ (3)

The given linear relationship allows for a simple and quick calculation of *α* and accordingly of the core body temperature. Furthermore, as the linear relationship is based on experimental validation, a practical and reliable approach for users is ensured.

In another embodiment, the processing unit is configured to determine the user's CBT based on a look-up table and/or a fitting function relating the estimated thickness to the CBT.

In an embodiment of the sensor device, the optical sensor comprises an optical transmitter, particularly a light-emitting diode, LED, (configured to transmit optical radiation (to and/or through the skin- and/or subcutaneous tissue)) and an optical receiver, particularly a photodiode or phototransistor, to detect transmitted light.

Preferably, the heat flux resistance of the insulating material of the insulating layer of the sensor device is pre-determined.

Preferably, the sensor device is a wearable device.

In an embodiment of the CBT estimation system, the optical sensor comprises an optical transmitter, particularly a light-emitting diode, LED, (configured to transmit optical radiation (to and/or through the skin- and/or subcutaneous tissue)) and an optical receiver, particularly a photodiode or phototransistor, to detect transmitted light.

Preferably, the optical radiation transmitted (i.e. irradiated/emitted) by the optical transmitter is radiation of broad white light and/or radiation in the vicinity of green, red or (near) infrared light, particularly of a specific wavelength.

As infrared light can penetrate deeper into the skin- and subcutaneous tissue than light in the visible spectrum, infrared light is particularly well-suited for measuring/determining the transmissive properties of said tissue.

In another embodiment, a distance between the optical transmitter and the optical receiver is between 0.7cm and 1.3cm, preferably between 0.8cm and 1.2cm, more preferably between 0.9cm and 1.1cm.

The distance defined allows for an accurate measurement, as the given distance ensures sufficient light transmission through tissue while minimizing errors. In particular, signal loss due to excessive scattering or absorption is minimized at the given distance.

In another embodiment of the CBT estimation system, the first temperature sensor and/or the second temperature sensor is a thermistor, particularly a negative temperature coefficient (NTC) thermistor.

NTC thermistors are a precise, cost-effective, and durable solution for body temperature measurement. Due to their high sensitivity, fast response time, and stability, they are particularly well-suited for medical sensors and wearable health devices.

In another embodiment, the CBT estimation system further comprises an insulating layer comprising a thermally insulating material arranged between the second temperature sensor and the first temperature sensor and configured to thermally isolate the first temperature sensor from the second temperature sensor.

Preferably the heat flow resistance of the insulating material is pre-determined.

This way, the measurement of the heat flux between the temperature sensors and thus the calculation of the CBT can be optimized, as the known heat flux resistance can be precisely integrated into the calculation.

In an embodiment of the CBT estimation system, the CBT estimation system is a wearable system and/or the CBT estimation system is arranged in a single housing.

The arrangement in a single housing, which can be a rigid housing or a flexible housing, has the advantage that the system is compact and easy to use, reducing complexity for users and healthcare providers. In particular, placement errors are eliminated, as both sensors measure from (almost) the same location so that a spot-like characterization of the skin- and subcutaneous tissue can be performed, improving measurement consistency. In fact, it is preferred that the optical sensor and the first temperature sensor are located as close to each other as possible. Preferably, the distance between said sensors is less than or equal to 10 mm, preferably less than or equal to 5 mm, more preferably less than or equal to 2 mm.

Preferably, if the optical sensor comprises an optical transmitter and an optical receiver, the first (and possibly the second) temperature sensor are arranged between the optical transmitter and the optical receiver. In other words, preferably, the optical path (transmission through tissue) should be close to (or even cross) the (first) temperature measurement spot.

Preferably, the first temperature sensor (i.e. user temperature sensor) is (solely) partially enclosed by the insulating material in order to allow for a direct contact with the user's skin. The second temperature sensor is preferably entirely enclosed by the insulating material (i.e. PE foam), which has the advantage that the second temperature sensor may not react on sudden changes of the environment / room temperature or air flows (e.g. caused by ventilation). Thus, the sensed temperature, though correlated with the environmental temperature, may differ from the real environmental (e.g. room) temperature.

An arrangement in a single housing can be seamlessly integrated into smartwatches, for example, and thus also enhances user comfort.

Preferably, in an embodiment of the CBT estimation system comprising the CBT estimation device, the optical sensor, the first temperature sensor, the second temperature sensor and the insulating layer, at least the optical sensor, the first temperature sensor, the second temperature sensor and the insulating layer are arranged in a sensor device as presented herein. Even if the CBT estimation device is separate from the rest of the system, but the remaining parts are arranged in a single (sensor) device, user comfort is enhanced.

In a further embodiment, the system further comprises a rendering unit configured to render visual and/or audible information in accordance with the CBT information comprising information about the estimated CBT.

The rendering unit may be a display unit, for example. It helps provide instant feedback for users or healthcare providers, allowing immediate action if needed. It thus simplifies usage in clinical and home environments, providing a user-friendly interface, e.g. for continuous monitoring.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a CBT estimation system for estimating a user's core body temperature according to the present invention;
Fig. 2 shows a schematic diagram of a second embodiment of a CBT estimation system for estimating a user's core body temperature according to the present invention;
Fig. 3A shows a schematic diagram of an embodiment of a sensor device according to the present invention as a cross-sectional view;
Fig. 3B shows a schematic diagram of the embodiment of the sensor device shown in Fig. 3A in a bottom view;
Fig. 4 shows a simplified heat flow representation between two layers in which a pair of temperature sensors is used to measure the heat flow across one of the layers;
Fig. 5 shows a schematic diagram of an embodiment of a CBT estimation device for estimating a user's core body temperature according to the present invention;
Fig. 6 shows a flow chart of an embodiment of a CBT estimation method according to the present invention;
Fig. 7A shows a schematic diagram of an experimental setup used to mimic skin- and subcutaneous tissue properties (above) and tables showing the relationship between the thickness of the skin- and subcutaneous tissue and a CBT offset (i.e. the deviation between the true CBT and the estimated CBT) as derived using said setup (below);
Fig. 7B shows alpha values for different skin- and subcutaneous tissue thicknesses at 37°C, 39°C and 41°C and an averaged alpha value for each thickness; and
Fig. 8 shows a schematical diagram of a setup for finding a relationship between thermal conductivity and optical transmission (above) and a diagram illustrating the relationship between the optical transmission and thickness of a user's skin- and subcutaneous tissue (below).

### DETAILED DESCRIPTION OF EMBODIMENTS

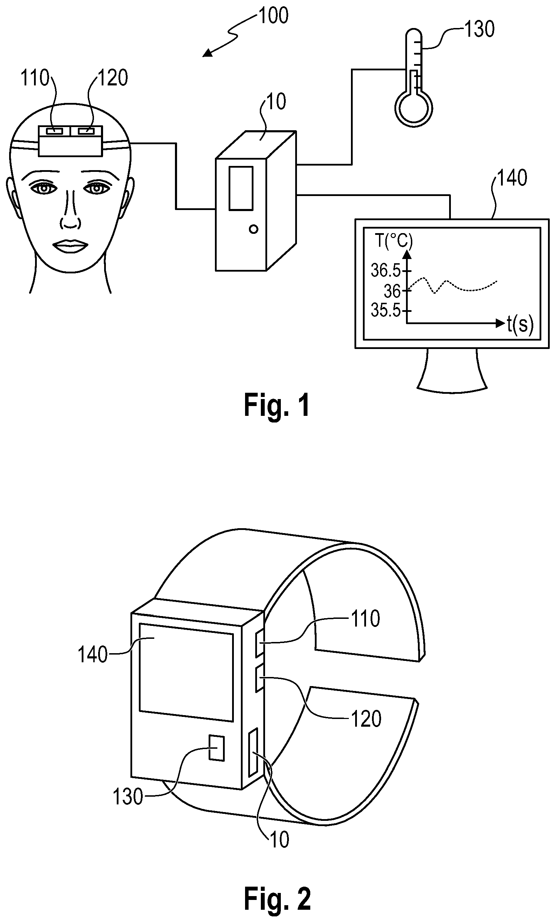

Fig. 1 shows a schematic diagram of a first embodiment of a CBT estimation system for estimating a user's core body temperature according to the present invention.

The CBT estimation system 100 comprises a CBT estimation device 10 for estimating a user's CBT, an optical sensor 110, and a first temperature sensor 120. In this embodiment, the CBT estimation system 100 further comprises a rendering unit 140. Optionally, the CBT estimation system 100 may further comprise a second temperature sensor 130 configured to measure the environmental temperature, i.e. the temperature of the (nearby) environment surrounding the CBT estimation system 100.

The optical sensor 110 and the first temperature sensor 120 are arranged within a single housing in this first embodiment of the CBT estimation system 100. In particular, the housing is configured as a (flexible) patch which can, for example, be attached to the user's forehead using straps around their head. However, other positions for placing the optical sensor 110 and the first temperature sensor 120 are conceivable. In general, any body part where arteries are close to the skin/surface, like the user's chest, are well-suited for placement of said sensors. Accordingly, said sensors are preferably configured to be attached to a body part where an artery is closed to the skin of the user. It is also conceivable, that said single "housing" is given by a plaster or other medical adhesive tape configured to be attached to the user's skin.

In this embodiment, the optical sensor 110 is configured to measure transmission of the user's skin- and subcutaneous tissue and to provide optical data comprising information about the optical transmission of the user's skin- and subcutaneous tissue and the first temperature sensor120 is configured to measure skin- and subcutaneous tissue temperature of the user and to provide first temperature data comprising information about the skin- and subcutaneous temperature the user.

The CBT estimation device 10 is configured to obtain (retrieve or receive) the optical data and the first temperature data from the optical sensor 110 and the first temperature sensor 120, respectively. Furthermore, the CBT estimation device 10 is configured to obtain second temperature data comprising information about an environmental temperature, e.g. from the second temperature sensor 130 if it is part of the CBT estimation system 100. However, the second temperature data may likewise be obtained from a database or the like. Using the optical data the CBT estimation device 10 may estimate a thickness of the user's skin- and subcutaneous tissue. Using this information, the first temperature data and the second temperature data the CBT estimation device 10 subsequently estimates the user's core body temperature (by using a pre-defined look-up table, for example). The CBT estimation device 10 then provides CBT information (comprising information about the estimated CBT) to the rendering unit 140 of the system 100.

The rendering unit 140 may then render the CBT information comprising information about the estimated CBT as visual information. Accordingly, in this embodiment, the rendering unit 130 is implemented as a display.

In general, the rendering unit 130 may be any means that outputs the CBT information in visual or audible form, e.g. in text form, as image or diagram, as sound or spoken words, etc. For instance, the rendering unit may be a display, a loudspeaker, a touchscreen, a computer monitor, the screen of a smartphone or tablet, etc.

In this first embodiment of the CBT estimation system 100, the CBT estimation device 10 is connected to the optical sensor 110, the first temperature sensor 120 and the rendering unit 140 (and optionally the second temperature sensor) via cable. However, other connections, particularly wireless connections, are conceivable.

Fig. 2 shows a schematic diagram of a second embodiment of a CBT estimation system for estimating a user's core body temperature according to the present invention.

The CBT estimation system 100 comprises a CBT estimation device 10 for estimating a user's CBT, an optical sensor 110, a first temperature sensor 120, an second temperature sensor 130 and a rendering unit 140. In this embodiment of the CBT estimation system 100, all components of the system are arranged in a single housing, for example in the housing of a smartwatch configured to be worn by the user or in the housing of a smartphone or similar device. The optical sensor 110 and the first temperature sensor 120 are arranged on the side of the housing which faces the user's skin when the CBT estimation system 100 is worn by the user. This way, contact of said sensor's to the user's skin can be ensured. On the other hand, the second temperature sensor 130 is preferably arranged on the opposite side of the housing to allow for a precise measurement of the environmental temperature unaffected by the user's CBT.

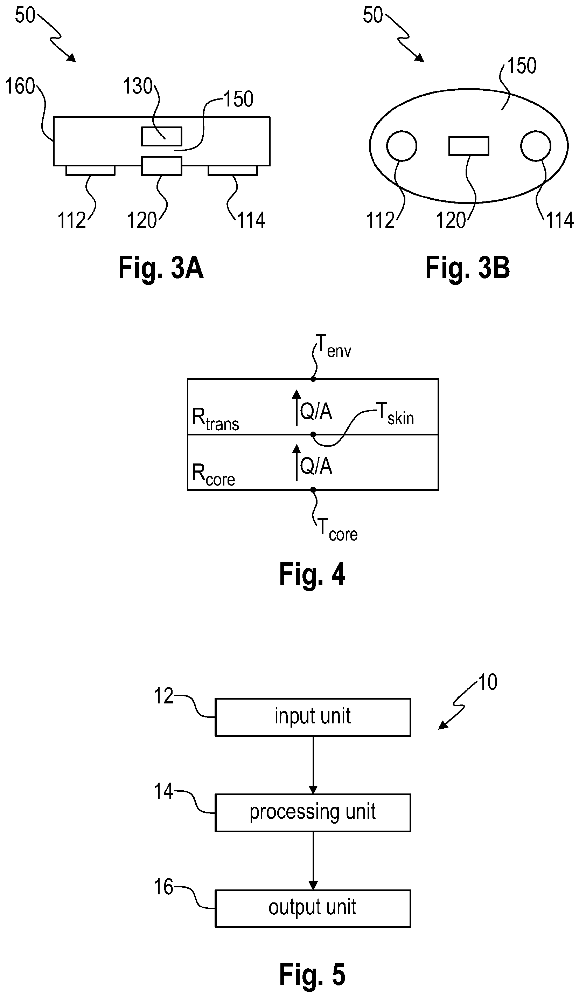

Fig. 3A shows a schematic diagram of an embodiment of a sensor device according to the present invention as a cross-sectional view.

The sensor device 50 comprises a patch 160 at least partially made of insulating material in which an second temperature sensor 130 (configured to measure a second temperature which is correlated with the environmental temperature), a first temperature sensor 120 (configured to measure the skin- and/or subcutaneous tissue temperature of the user), and an optical sensor 110 comprising an optical transmitter 112 and an optical receiver 114, are - at least partially - embedded (in the embodiment shown the second temperature sensor is completely enclosed by the insulating material). The second temperature sensor 130 and the first temperature sensor 120 are arranged in such a manner, that they are thermally separated by an insulating layer 150 of the insulating material of the patch 160. The insulating material may comprise polyethylene (PE) foam (with a thermal conductivity of about 0.035 W/mK), for example.

The basic principle of the sensor device 50 is the combination of an indirect core body temperature sensor represented by the first temperature sensor 120 and the second temperature sensor 130, i.e. a "single heat flux device", with an optical sensor 110. The optical transmitter 112 is configured to irradiate the skin- and/or subcutaneous tissue of the user with an optical signal (e.g., having broad white light or a specific wavelength, e.g., near infrared light) and the optical receiver 120 is configured to receive the optical signal (having travelled through the skin- and/or subcutaneous tissue) which is damped as a function of the optical skin- and/or subcutaneous tissue characteristics, from which a (thermal) characteristic of the skin- and/or subcutaneous tissue can be derived which finally can be used to improve the CBT measurement of a user.

For example, using the sensor device 50 an optical transmission measurement of the skin- and subcutaneous tissue of the user may be performed from which conclusions on the patient-physiology can be made from which then a user-specific heat transfer function (or parameter) may be derived to improve a CBT measurement.

Fig. 3B shows a schematic diagram of the embodiment of the sensor device shown in Fig. 3A in a bottom view.

As can be seen from Fig. 3B, in this embodiment the patch 160 has a round shape. However, other shapes, such as a rectangular shape, are conceivable as well.

Fig. 4 shows a simplified heat flow representation between two layers in which a pair of temperature sensors is used to measure the heat flow across one of the layers. The lower layer with heat flux resistance (thermal resistance) *R_{core}* shall represent the tissue between the core body area and the outer surface of the skin, while the upper layer with heat flux resistance *Rₜᵣₐₙₛ* shall represent a thermally insulating material on top of the skin. The temperature sensors are placed in two locations: one sensor near, preferably on, the skin, configured to measure *Tₛₖᵢₙ* (the bottom or skin temperature) and a second sensor on top of the insulating material, configured to measure *Tₑₙᵥ* , the environmental temperature.

The heat flow starting from the core body temperature *T_{core}*, which is the temperature to be estimated, outwards can be modeled based on a linear flow as: ${T}_{core} = {T}_{skin} + \frac{{R}_{core}}{{R}_{trans}} ⋅ \left({T}_{skin}-{T}_{env}\right) .$ (4)

As can be seen from the given formula, to estimate the CBT correctly, the value of *R_{core}* needs to be known as precisely as possible. However, this parameter is basically a combination of tissue properties, which is user-dependent and varies also with location, and the thermal contact between temperature sensor and skin surface.

Fig. 5 shows a schematic diagram of an embodiment of a CBT estimation device for estimating a user's core body temperature according to the present invention. The CBT estimation device 10 may e.g. be implemented as or in a processing unit or computer, in software and/or hardware.

The CBT estimation device 10 comprises an input unit 12 configured to obtain first temperature data comprising information about a skin- and/or subcutaneous tissue temperature of the user, second temperature data comprising information about an environmental temperature, and optical data comprising information about an optical transmission and/or absorption of the user's skin- and/or subcutaneous tissue, i.e. about properties of the skin- and or subcutaneous tissue relating to optical transmission and/or absorption. The input unit 12 may be directly coupled or connected to any one of a user interface configured to obtain user input, e.g. second temperature data, a database comprising the data to be obtained, and/or one or more of a first temperature sensor 120, an second temperature sensor 130, and an optical sensor 110, or may obtain (i.e. retrieve or receive) these data from a storage, buffer, network, or bus, etc. The input may thus e.g. be a (wired or wireless) communication interface or data interface, such as a Bluetooth interface, WiFi interface, LAN interface, HDMI interface, direct cable connect, or any other suitable interface allowing signal transfer to the CBT estimation device 10.

The CBT estimation device 10 further comprises a processing unit 14 configured to estimate a thickness of the user's skin- and/or subcutaneous tissue based on the optical data and to estimate the user's CBT based on the first temperature data, the second temperature data and the estimated thickness.

The processing 14 may be any kind of means configured to process the data obtained and to estimate a thickness of the user's skin- and/or subcutaneous tissue based on the optical data and to estimate the user's CBT based on the first temperature data, the second temperature data and the estimated thickness. It may be implemented in software and/or hardware, e.g. as a programmed processor or computer or app on a user device such as a smartphone, smartwatch, tablet, laptop, PC, workstation, etc.

The output unit 16 of the CBT estimation device 10 is configured to provide, particularly output, CBT information comprising information about the estimated CBT, particularly to a rendering unit 140 configured to render audible and/or visual information in accordance with the CBT information. The output unit 16 may generally be any interface configured to provide the CBT information, e.g. transmits it to another device or provides it for retrieval by another device (e.g. a smartphone, computer, tablet, etc.). It may thus generally be any (wired or wireless) communication or data interface.

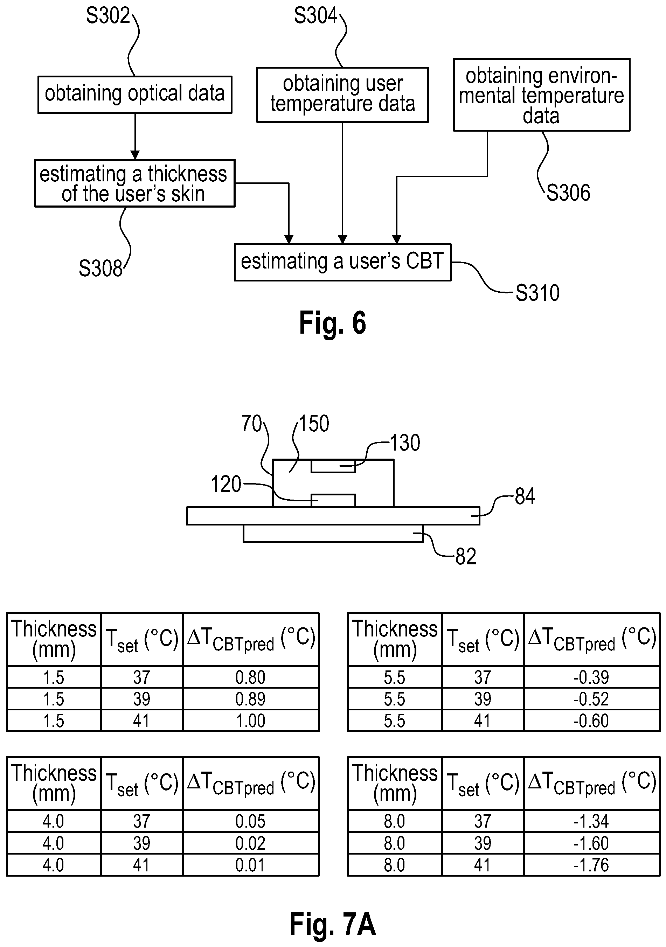

Fig. 6 shows a flow chart of an embodiment of a CBT estimation method according to the present invention.

In a first step S302 optical data comprising information about transmission and/or absorption of the user's skin- and/or subcutaneous tissue are obtained (and provided). In a second step S304 first temperature data comprising information about a skin- and/or subcutaneous tissue temperature of the user are obtained (and provided). In a third step S306 second temperature data comprising information about an environmental temperature are obtained (and provided). The order of the steps S302, S304, and S306 is irrelevant for the CBT estimation method.

In a fourth step S308 a thickness of the user's skin- and/or subcutaneous tissue is estimated based on the optical data and in a fifth step S310 the user's CBT is estimated based on the first temperature data, second temperature data and the estimated thickness.

Fig. 7A shows a schematic diagram of an experimental setup used to mimic skin- and subcutaneous tissue properties (above) and tables showing the relationship between the thickness of the skin- and subcutaneous tissue and a CBT offset (i.e. the deviation between the true CBT and the estimated CBT) as derived using said setup (below).

The setup used is based on the finding that a silicon layer of 4.5mm thickness adequately represents the thermal characteristics of skin- and subcutaneous tissue of an "averaged" human body.

The setup shows a silicone layer 84 of pre-determined thickness, mimicking a pre-determined skin/tissue thicknesses, put on top of a heat plate 82 of pre-determined temperature (so called "set point"), mimicking the CBT. A CBT sensor 70 is arranged on top of the silicone layer 84, the CBT sensor 70 comprising a "first temperature sensor 120" configured to measure the temperature of the (surface) of the silicone layer 82 (i.e. the "skin- and subcutaneous tissue") and an second temperature sensor 130 configured to measure the temperature of the environment, both sensors being separated by a thermally insulating layer 150.

Using equation (4) the CBT can be estimated using an "averaged" α value (*α_{average} =* 0.3110) and a corresponding CBT offset from the given set point (i.e. the correct measured temperature of the heat plate 82) can be determined.

The tables in the lower part of Fig. 7A show as function of the layer thickness (left column) and the temperature set point of the heat plate (mid column) the determined offset, i.e. deviation, from this set point (right column), based on the actual temperature measurement and using the averaged α value. The tables particularly show the CBT as measured and a deviation between the measured CBT and the estimated CBT (using the given fixed value of alpha) for four different layer-thicknesses (1.5 mm, 4 mm, 5.5 mm and 8 mm) and three different temperatures (37 °C, 39 °C and 41 °C).

Not surprisingly, for a 4 mm thick silicone layer the predicted CBT almost matches the set point of the heat plate. However, the farer away from the 4 mm the worse the estimated, non-corrected CBT predictions become. In particular, for obese patients (represented by a silicone layer thickness of 8mm) an offset of -1.76 °C would apply (if the set point was 41°C), which could result into a severe medical miss-treatment, because the patient would be at a temperature of already 41 °C, while the CBT sensor would indicate a temperature of only 39.24 °C.

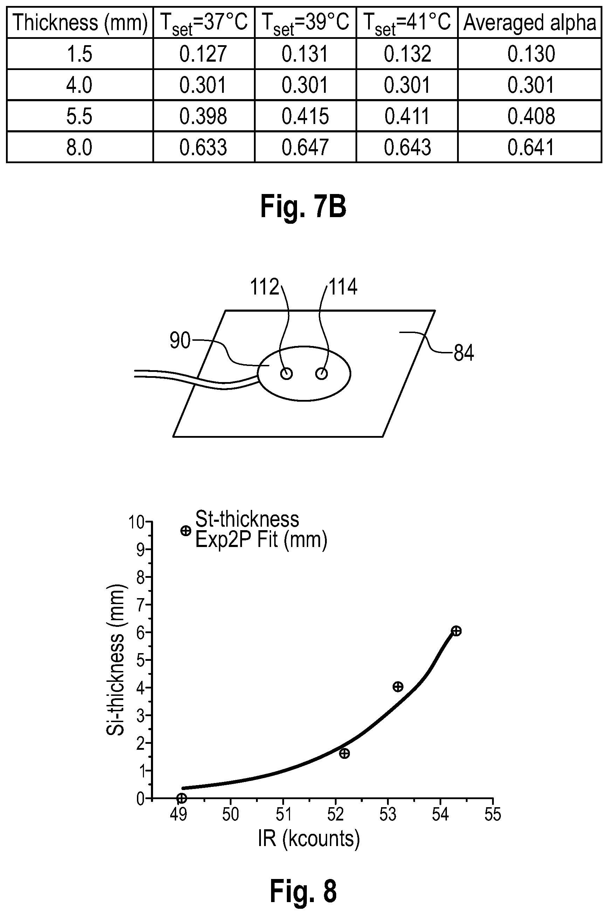

These results can be used to find a relationship between (the true) alpha and the thickness of a user's skin- and subcutaneous tissue. In fact, as can be seen from Fig. 7B, the above results allow for a corrected alpha, optimized for each of the given settings/configurations described with respect to Fig. 7A.

Fig. 7B shows alpha values for different skin- and subcutaneous tissue thicknesses at 37°C, 39°C and 41°C and an averaged alpha value for each thickness.

From the table shown in Fig. 7B a simple averaged linear correlation can be derived, approximating the alpha value as a function of the silicone thickness, mimicking patient skin/tissue thickness d: $α = 0.0782 ∗ d - 0.0017 .$ (5)

As outlined above, silicone layers of different thicknesses can be used to mimic thermal transmission (or conductivity) of different skin/tissue thicknesses.

In a further experiment the optical transmissions of different silicone layer thicknesses have been identified to derive a relationship between thermal conductivity (and thus the thickness) of the user's skin- and subcutaneous tissue and optical transmission of the tissue. For this, another experimental setup as shown in Fig. 8 has been used.

Fig. 8 shows a schematical diagram of a setup for finding a relationship between thermal conductivity and optical transmission (above) and a diagram illustrating the relationship between the optical transmission and thickness of a user's skin- and subcutaneous tissue (below).

The setup comprises an optical transmitter 112 and an optical receiver 114, which are embedded in an optical patch 90, the optical patch 90 being arranged on top of a silicone layer 84 mimicking the skin- and subcutaneous tissue. The optical transmitter 112 is configured to irradiate red- and/or infra-red light and the optical receiver 114 is configured to receive this light (optical waves). In the shown setup the optical transmitter 112 and the optical receiver 114 are spaced apart by about 1 cm.

The irradiated light enters the silicone layer 84 underneath the optical transmitter 112, travels through the silicone layer 84 and is received by the optical receiver 114. While traveling through the silicone layer 84, the optical waves are damped because of intrinsic absorption effects of the material. The received signal intensity (received e.g. via a photodiode with connected electrical amplifier and an analogue-digital converter) is a direct measure of the thickness of the medium (in this case the silicone layer 84 but in the end also the thickness of the skin/tissue of the user).

Since the signal losses due to traveling through the silicone are significantly higher than the losses due to a reflection at its surface (when the optical signal enters the medium), a variation of the color of the medium (which would represent a different user skin color) does not significantly impact the signal intensity received by the optical receiver 114. Reflection/absorption can further be minimized by choosing specific wavelengths, e.g. in the (near) infra-red regime. Accordingly, a change of (skin) color/tone may be neglected.

The lower part of Fig. 8 shows a diagram of the silicone layer thickness (mimicking different skin thicknesses) as a function of the received signal intensity, represented as counts (of an AD converter). As can be seen from the diagram, there is a clear non-linear correlation between silicone layer thickness and received intensity. In particular, as can be seen from the diagram, with an increased thickness of the silicone layer the width/height of the optical channel is increased. Thus, the higher the thickness, the higher is the number of counts. By means of this optical characteristic each skin/tissue thickness can be correlated to a specific value of alpha, which finally allows to improve the CBT precision because of being user specific.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Core body temperature, CBT, estimation device (10) for estimating a user's CBT, the CBT estimation device (10) comprising:
- an input unit (12) configured to obtain:
- first temperature data comprising information about a skin- and/or subcutaneous tissue temperature of the user,
- second temperature data comprising information about a second temperature, and
- optical data comprising information about an optical transmission and/or absorption of the user's skin- and/or subcutaneous tissue;
- a processing unit (14) configured to estimate a thickness of the user's skin- and/or subcutaneous tissue based on the optical data and to estimate the user's CBT based on the first temperature data, the second temperature data and the estimated thickness, and
- an output unit (16) configured to provide CBT information comprising information about the estimated CBT.

2. CBT estimation device (10) according to claim 1,
wherein the processing unit (14) is further configured to determine a user-specific heat transfer function based on the estimated thickness and to estimate the user's CBT based on the first temperature data, the second temperature data and the determined heat transfer function.

3. CBT estimation device (10) according to claim 2,
wherein the processing unit (14) is configured to determine the heat transfer function based on a look-up table and/or a fitting function relating the estimated thickness to the heat transfer function.

4. CBT estimation device (10) according to claim 3,
wherein the relationship between the estimated thickness and the heat transfer function is a linear relationship.

5. CBT estimation device (10) according to any one of the preceding claims,
wherein the processing unit (14) is configured to determine the user's CBT based on a look-up table and/or a fitting function relating the estimated thickness to the CBT.

6. Sensor device (50) for measuring optical and thermal features of a user's skin- and/or subcutaneous tissue, the sensor device (50) comprising:
- a second temperature sensor (140) configured to measure environmental temperature;
- a first temperature sensor (120) configured to measure the skin- and/or subcutaneous tissue temperature of the user;
- an optical sensor (110) configured to measure transmission and/or absorption of the user's skin- and/or subcutaneous tissue; and
- an insulating layer comprising a thermally insulating material,
wherein the insulating layer is arranged between the second temperature sensor (130) and the first temperature sensor (120) and configured to thermally isolate the first temperature sensor (120) from the second temperature sensor (130).

7. Core body temperature, CBT, estimation system (100) for estimating a user's CBT, the CBT estimation system (100) comprising:
- a CBT estimation device (10) as claimed in any one of the preceding claims; and any one of the following:
- an optical sensor (110) configured to measure transmission and/or absorption of the user's skin- and/or subcutaneous tissue,
- a first temperature (120) configured to measure skin- and/or subcutaneous tissue temperature of the user, and
- a second temperature sensor (130) configured to measure the environmental temperature.

8. CBT estimation system (100) according to claim 7,
wherein the optical sensor (110) comprises an optical transmitter (112), particularly a light-emitting diode, LED, and an optical receiver (114), particularly a photodiode or phototransistor to detect transmitted light.

9. CBT estimation system (100) according to claim 8,
wherein a distance between the optical transmitter and the optical receiver is between 0.7cm and 1.3cm, preferably between 0.8cm and 1.2cm, more preferably between 0.9cm and 1.1cm.

10. CBT estimation system (100) according to any one of claims 7 to 9,
wherein the first temperature sensor (120) and/or the second temperature sensor (130) is a thermistor, particularly a negative temperature coefficient thermistor.

11. CBT estimation system (100) according to any one of claims 7 to 10,
further comprising an insulating layer comprising a thermally insulating material arranged between the the second temperature sensor (130) and the first temperature sensor (120) and configured to thermally isolate the first temperature sensor(120) from the second temperature sensor (130).

12. CBT estimation system (100) according to claim 10,
wherein the CBT estimation system (100) is a wearable system and/or wherein the CBT estimation system is arranged in a single housing.

13. CBT estimation system (100) according to any one of claims 7 to 12,
further comprising a rendering unit (140) configured to render visible and/or audible information in accordance with the CBT information comprising information about the estimated CBT.

14. Core body temperature, CBT, estimation method for estimating a user's CBT, the CBT estimation method comprising:
- obtaining first temperature data comprising information about a skin- and/or subcutaneous tissue temperature of the user;
- obtaining second temperature data comprising information about a second temperature;
- obtaining optical data comprising information about transmission and/or absorption of the user's skin- and/or subcutaneous tissue,
- estimating a thickness of the user's skin- and/or subcutaneous tissue based on the optical data;
- estimating the user's CBT based on the first temperature data, second temperature data and the estimated thickness; and
- providing CBT information, comprising information about the estimated CBT.

15. Computer program comprising program code means for causing a computer to carry out the steps of the method as claimed in claim 14 when said computer program is carried out on the computer.
